# EUROPEAN PATENT APPLICATION

(11) **EP 3 730 147 A1**
(43) Date of publication of application: **28.10.2020**
(21) Application number: 20460016.7
(22) Date of filing: 23.03.2020
(51) Int. Cl.: A61K 36/185, A61K 31/192, A61P 3/10

(54) **PREPARATION WITH HYPOGLYCEMIC EFFECT**

(30) Priority: 24.04.2019 PL 42972619
(71) Applicant: Instytut Biotechnologii i Badan Medycznych "Biolamed" SP. Z O. O., 15-430 Bialystok (PL)
(72) Inventor: TOMULEWICK, Mikolaj, 15-507 Bialystok (PL)
(74) Representative: Dobkowska, Danuta Elzbieta

(57) **Abstract**

The subject of the invention is a preparation with hypoglycemic effect, used adjunctively in patients with diabetes, and having an impact on the activity of carbohydrate metabolizing enzymes and on the expression profile of the genes of the enzymes involved in carbohydrate metabolism.

Preparation with hypoglycemic effect containing as active substance a compound from the group of lupane triterpenoids isolated from the birch bark Betula pendula Roth. characterized in that lupeol contains 0.5-24.5% w/w, betulin contains 0.5-24.5% w/w, betulinic acid contains 0.5-24.5% w/w, betulonic acid contains 0.5-24.5% w/w.

The preparation is characterized by the fact that the active substances are enclosed in a 500 or 800 mg gelatin capsule.

## Description

The subject of the invention is a preparation with hypoglycemic effect, used adjunctively in patients with diabetes, and having an impact on the activity of carbohydrate metabolizing enzymes and on the expression profile of the genes of the enzymes involved in carbohydrate metabolism.

Epidemiological data from 2018 indicate that every year 5 million people die from diabetes and its complications. By 2040, it is estimated that 642 million people in the range of 20-79 age will have diabetes. In Poland, by 2015, there were 3 million adults with diabetes. It is expected that by 2040 this number will increase to over 4 million people.

With the increased numer of patients, the treatment costs will increase as well. Diabetes is not only a disease that consumes a lot of financial resources, but also causes the social costs of so-called non-medical due to employee absences, a decrease in their productivity, and as a consequence premature resignation from professional activity [Report of the Institute of Health Protection, 2018]. Considering the above, the goal of this invention is to create a dietary supplement, a widely available agent supporting the reduction of blood glucose, which will also support the prevention of diabetes development in Poland.

Diabetes is a metabolic disease with a genetic predisposition, accompanied by chronic hyperglycaemia due to absolute or relative insulin deficiency. It is one of the most important medical and social problems of our time, because it occupies a high place in the structure of chronic pathology of all age groups and is one of the most important reasons for the increase in disability and mortality both in Poland and in the world.

Metabolic disorders occurring in the course of diabetes can lead to serious and irreversible changes in the body. Hyperglycaemia plays a major role in this process. The incidence of diabetes in epidemiological studies is about 6% of the total population, with the majority of people with diabetes being of working age.

The World Health Organization (WHO) distinguishes: type 1 diabetes, type 2 diabetes, MODY type diabetes, gestational diabetes and other specific types diagnosed based on fasting and postprandial blood glucose.

A common feature of all types of diabetes is the continuous increase blood glucose level, which in turn leads to the development of metabolic disorders in tissues. Elevated glucose level in the blood and body fluids leads to many pathological changes in various organs and body systems causing serious complications such as acute ketoacidosis, lactic acidosis, hypoglycemia, hyperosmolar coma as well as nephro-, ophthalmic, retino- and encephalopathy [American Diabetes Association, 2014].

The main and effective drug used in the treatment of type 1 diabetes are insulin injections, while in type 2 diabetes - oral hypoglycemic agents such as: biguanides, sulfonylureas, glinides, thiazolidinediones, α-glycosidase inhibitors as well as replacement insulin therapy in the ineffective use of oral drugs. Hypoglycemic drugs can be considered as adjuvant (complementary) therapy aimed at reducing the risk of complications, which is a consequence of constant hyperglycemia.

When using known hypoglycemic agents, many contraindications should be borne in mind: cardiovascular insufficiency, respiratory system, ketoacidosis and lactic acidosis, pregnancy, breastfeeding, as well as gastrointestinal side effects (nausea, vomiting, diarrhea) as well as endocrine (hypoglycaemia) and hematopoietic system.

Currently, the target of diabetes pharmacotherapy is to achieve stable compensation, prevent the development of complications and look for prevention methods to development of this disease entity. Given the relative limitations and the significant number of side effects of synthetic drugs, supporting therapy with natural preparations still poses a technological challenge in the biotechnology and pharmaceutical market.

From the US patent US 20160166631 is known a preparation containing in its composition, among others magnesium, zinc, chromium, vitamin B12, American ginseng, cinnamon bark, lutein and green tea extract, used as a dietary supplement to control blood glucose levels and how to prevent, alleviate or treat type 2 diabetes.

From the international patent WO 2019008551 is known a composition with anti-diabetic effect enriched with triterpenoids from the extract of ossicular, a medicinal plant originating in Mexico known as spiral ginger, for the treatment of diabetes.

From US patent US 20170182110 is known a composition for the prevention or treatment of diabetes, comprising the extract of the leaves of the hybrid plant Camellia sinensis and Camellia taliensis.

One of the more promising groups of compounds are triterpenoids and their derivatives, mainly lupane triterpenoids. Triterpenes belong to the group of organic compounds. They are isoprene hexamers. Years of experience have allowed to describe over four thousand triterpenes and their derivatives. Betulin and its derivatives belong to the group of pentacyclic triterpenes characterized by the presence of five six-carbon or one five- and four six-carbon rings. The skeleton is made of six isoprene units and is formed as a result of cyclization of the squalene molecule. Betulin and its derivatives can be isolated from birch bark by sublimation by extraction with organic solvents (dichloromethane, chloroform, acetone) and crystallization from appropriate solvents. Betulin together with other triterpenoids such as lupeol, betulinic acid, betulinic aldehyde and allobetulin are found in large quantities in the birch bark.

One of the natural sources of lupane triterpenoids is the outer layer of the bark of white birch species (Betula) e.g. B. verrucosa, B. pendula, B. pubescens, B. alba. Birch bark is a byproduct of wood processing and is practically not used in economic activity. According to various sources, birch bark contains up to 40% betulin and up to 5-10% lupeol. Birch bark extracts contain betulin and oxidized derivatives: betulinic acid, betulinic aldehyde, betulinic acid methyl ester, betulonic aldehyde, betulonic acid, as well as a large number of related compounds.

The purpose of the invention is to broaden the range of preparations of natural origin with hypoglycemic effect on the activity of carbohydrate metabolizing enzymes and on the expression profile of the genes of the enzymes involved in carbohydrate metabolism.

A preparation of natural origin with a hypoglycemic effect, according to the invention, containing as active substance compounds belonging to the group of lupane triterpenoids isolated from the birch bark Betula pendula Roth. It is characterized by the fact that as active ingredients it contains 0.5-24.5% lupeol, 0.5-24.5% betulin, 0.5-24.5% betulinic acid, 0.5-24.5% betulonic acid, and the active substance is enclosed in a gelatin capsule in an amount of 500 or 800 mg. The pharmaceutical carrier is selected from the group consisting of polyvinylpyrrolidone, cellulose derivatives, including hydroxypropylmethylcellulose and hydroxypropylcellulose, cyclodextrin, gelatin, hypromellose phthalate, sugars and polyhydric alcohols, calcium sulfate dihydrate, dibasic calcium phophate, sorbitol, mannitol, anhydrous lactose, dextrose, acacia, tragacanth, ethylcellulose, methylcellulose, polyvinyl alcohol, sodium carboxymethylcellulose, silicon dioxide, polyethylene glycol 400, polyethylene glycol 4000, starch, porous silica carriers, aluminum silicate, calcium silicate, sugars, magnesium stearate, cellulose, calcium phosphate and combinations thereof. The pharmaceutical carrier is modified starch.

The preparation of natural origin with hypoglycemic effect, according to the invention, causes a hypoglycemic effect by lowering blood glucose concentration, and also improves morphometry of pancreatic islands and increases insulin concentration in animals with induced diabetes.

The present invention will be described in the examples with reference to a particular preferred embodiment, which does not limit other possible solutions according to the invention.

### In vitro investigation of the mechanisms of hypoglycemic action of lupane terpenoids

Rat liver glycogen phosphorylase, rat liver and skeletal muscle hexokinase, rat liver glucose-6-phosphate dehydrogenase (DG-6-P), rat liver and skeletal muscle phosphofructokinase were selected as potential targets for *in vitro* testing of the hypoglycemic activity of lupane triterpenoids.

Investigating the effect of lupane triterpenoids on glycogen phosphorylase activity, a reduced enzyme activity in rat liver homogenates and post-mitochondrial fraction was demonstrated in all study groups. Betulin, betulon and betulinic acids had the greatest inhibitory effect.

Tested compounds did not significantly affect hexokinase activity in both liver and skeletal muscle of rats.

Surprising results were obtained by examining the effect of lupane triterpenoids on glucose-6-phosphate activity. A 10% increase in enzyme activity was observed in rat liver homogenates, whereas the opposite effect was observed in the post-mitochondrial fraction. In both cases, the observed effects were dose-dependent. Since no definitive response could be obtained regarding the effect of lupane triterpenoids on DG-6-P activity, an additional enzyme purification procedure was performed and all compounds used were shown to inhibit enzyme activity in a dose-dependent manner.

**Table 1. Effect of lupane triterpenoids on DG-6-P activity in 5.6-fold purified rat liver**

| Group | Lupane triterpenoids concentration | | | |
|---|---|---|---|---|
| | 5 µM | | 25 µM | |
| | E | % of control | E | % of control |
| Control | 0,210±0,015 | - | 0,212±0,015 | - |
| Betulin | 0,149±0,004 | 71,00±2,02 | 0,100±0,002 | 47,60±0,97 |
| 3,28-betulin diacetate | 0,158±0,002 | 75,00±1,18 | 0,111±0,008 | 52,80±3,81 |
| Lupeol | 0,143±0,005 | 68,00±2,38 | 0,113±0,009 | 53,60±4,23 |
| Betulinic acid | 0,144±0,002 | 68,80±0,86 | 0,093±0,007 | 44,20±3,27 |
| Betulonic acid | 0,148±0,003 | 70,35±1,29 | 0,109±0,007 | 51,90±3,18 |
| Allobetulin | 0,151±0,004 | 72,00±1,92 | 0,103±0,002 | 48,95±0,54 |

Lupane triterpenoids at the tested concentrations showed a multidirectional effect on phosphofructokinase activity in homogenates and post-mitochondrial fraction in both liver and muscle tissue of rats. Probably the tested lupane triterpenoids did not directly affect the enzyme itself, but mediated their effect on phosphofructokinase by acting on other cellular enzymes, changing the concentrations of allosteric regulators (ATP, AMP, etc.).

Therefore, there are premises to assume that the hypoglycemic effect of lupane triterpenoids may result from a change in the regulation of glucose homeostasis at the level of glycogen degradation in the liver and muscle tissue, as well as from a change in the direction of intracellular glucose metabolism in the liver from gluconeogenesis to glycolysis.

### Effect of lupane terpenoids on insulin secretion by β-cells of the Langerhans islets of the pancreas

To study the effect of betulin and betulinic acid on insulin secretion by pancreatic islet β-cells, their solutions in DMSO were incubated with isolated Langerhans islands in concentrations of 10 µM for 30 minutes, after which the insulin content in the incubation medium was determined by enzyme immunoassay.

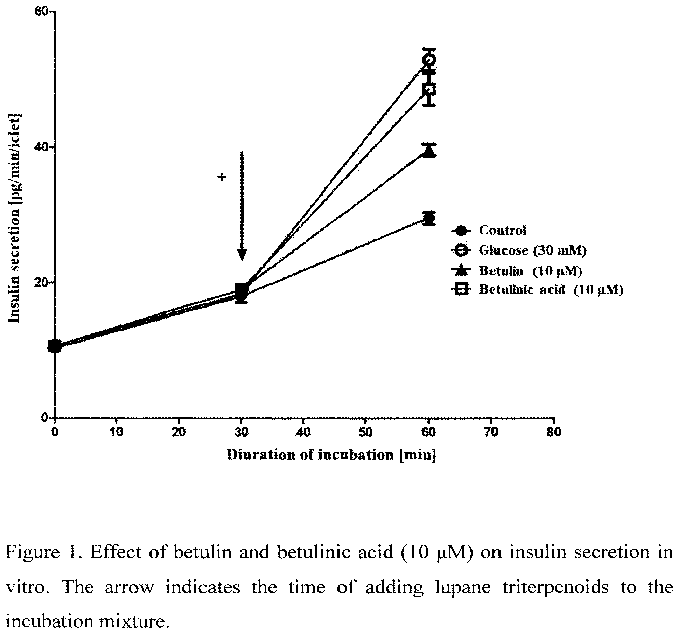

Betulin and betulinic acid have been shown to statistically significantly increase insulin secretion by pancreatic islet β-cells compared to the control group. The strongest reaction was observed after 30 minutes of incubation in the presence of 10 µM lupane triterpenoids (Figure 1).

### Assessment of the effect of lupane triterpenoids on blood glucose levels in rats with induced hyperglycemia

To study the effect of betulin, 3,28-diacetate betulin, betulinic acid and lupeol on blood glucose levels in rats with induced hyperglycemia, the animals received 2 hours prior to glucose administration, test substances at doses of 100 or 1000 mg/kg. Hyperglycaemia was caused by oral administration of a 40% glucose solution at a dose of 2 g/kg (OGTT-2) or 4 g/kg (OGTT-4).

The study was conducted on Wistar rats weighing 220-250 g. All animals were subjected to two weeks of observation prior to the experiment to exclude infection. Rats were housed in standard cages, 5 individuals in each. The animals stayed in a room with a temperature of 22±2°C, air humidity 60-70% and in natural lighting (12/12 in a day/night cycle). The animals had free access to water and standard laboratory feed ad libitum.

Animals were randomized into 5 groups, 8 individuals each.

Hyperglycaemia was caused by administration of a 40% glucose solution at doses of 2 or 4 g/kg. Animals from the respective groups received triterpenoids suspensions in 2% starch, once, intragastrically, at 100 and 1000 mg/kg, 2 hours before glucose administration.

Blood glucose was determined with a Roche AccuCheck Active glucometer (USA) before and after glucose administration every 30 minutes for 2 hours and then every 60 minutes for 6 hours.

The tested lupane terpenoids have been shown to cause a statistically significant and dose dependent reduction in glucose levels in animals with induced hyperglycemia, but no increase in this effect has been observed after the 1000 mg/kg dose.

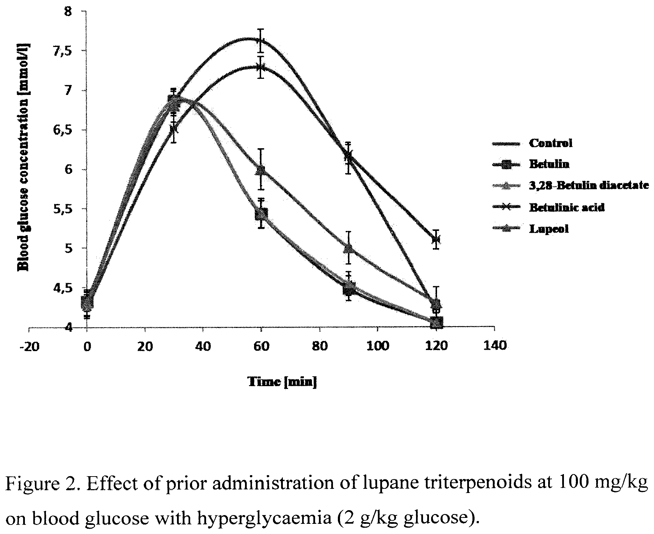

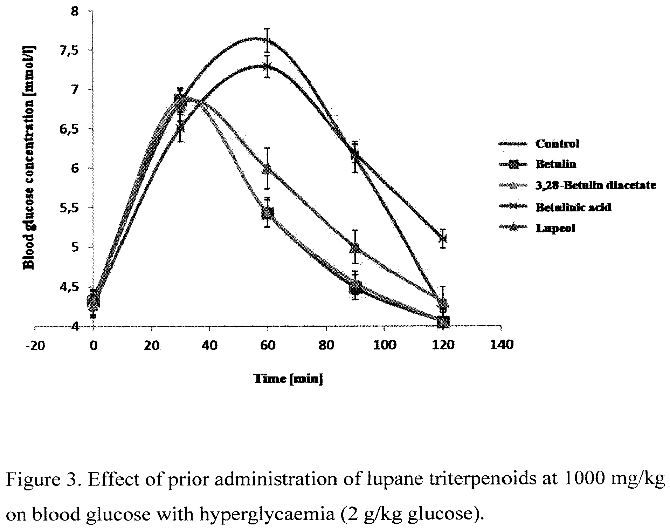

The most significant reduction in blood glucose levels at the peak of hyperglycemia by 30-35% compared to control animals observed after administration of betulin or betulinic acid (Figure 2 and 3).

### Study of the hypoglycemic effect of lupane triterpenoids in experimental diabetes in rats

The study was conducted on Wistar rats weighing 220-250 g. All animals were subjected to two weeks of observation prior to the experiment to exclude infection. Rats were housed in standard cages, 5 individuals in each. The animals stayed in a room with a temperature of 22±2°C, air humidity 60-70% and in natural lighting (12/12 in a day/night cycle). The animals had free access to water and standard laboratory feed ad libitum.

Diabetes was caused by a single intraperitoneal administration of alloxan at a dose of 170 mg/kg bw. The development of diabetes was observed on the basis of the main symptoms (polydipsia, polyphagia), glycosuria and hyperglycemia. Animals with high glucose levels of at least 14 mmol/L were selected for the experiment.

Animals were randomized into 6 groups of 8 individuals each. The first group, the control group, was formed from healthy animals without diabetes. The control group (healthy animals) received NaCl 1 ml/kg; Group 2. - diabetes mellitus (DM), received NaCl 1 ml/kg; Group 3. - DM + Betulin, at a dose of 100 mg/kg; Group 4. - DM + Lupeol, at a dose of 100 mg/kg; Group 5 - DM + Betulonic acid, at a dose of 100 mg/kg; Group 6 - DM + Betulinic acid, at a dose of 100 mg/kg; Group 7 - DM + Betulin-3,28-diacetate, at a dose of 100 mg/kg.

4 hours after drug administration, all animals intragastrically received a 40% glucose solution at a dose of 4 g/kg. The time of administration is determined in the available literature on its pharmacokinetics - peak plasma levels of lupane triterpenoids in rats are observed 3-4 hours after intragastric administration.

Peripheral blood glucose was determined using a Roche AccuCheck Active meter (USA) before and after glucose administration every 60 minutes for 5 hours. A drop of blood was taken from the tail tip incision.

In animals with diabetes and induced hyperglycemia, it was shown that the initial administration of test compounds was accompanied by a significant reduction in blood glucose levels compared to the control group of animals with alloxan-induced diabetes (Table 2). It should be noted that in the group of animals with diabetes without treatment (DM), the glucose level did not approach the lower limit even 5 hours after the glucose load, and in the DM + Betulin and DM + Betulinic acid groups the indicators normalized, as in healthy animals, already 4 hours after glucose administration.

**Table 2. Blood glucose concentration in rats with alloxane-induced diabetes (mmol/L).**

| Blood collection time, hours | Glucose concentration, mmol/l | | | | | |
|---|---|---|---|---|---|---|
| | Diabetes | Diabetes+ Betulin | Diabetes+ Lupeol | Diabetes+ Betulinic acid | Diabetes+ Betulonic acid | Diabetes+ 3,28-Betulin-3,28-diacetate |
| 0 | 10,38±0,7 8 | 10,82±0,31 | 10,71±0,6 3 | 10,12±0,70 | 10,32±0,77 | 11,77±0,7 0 |
| 2 | 27,21±0,7 7 | 22,61±0,4* | 26,50±1,2 0 | 25,64±1,50 | 18,5±0,32** | 26,22±1,6 0 |
| 5 | 13,48±0,7 9 | 6,01±0,78** | 9,38±0,75 * | 7,88±1,30* * | 6,52±0,7** | 8,51±0,52 * |
| * - p< 0,05 compared to the Diabetes group | | | | | | |
| ** - p< 0,01 compared to the Diabetes group | | | | | | |

### Evaluation of the functional state of the pancreas in modeling diabetes in laboratory animals

The possible cytoprotective effect of lupane triterpenoids on the functional state of the pancreas in alloxan-induced diabetes in rats was evaluated.

Alloxane was used to induce diabetes, which causes damage to the necrotic islet type pancreatic apparatus due to selective accumulation in β-cells and induction of massive free radical generation.

The study was conducted on Wistar rats weighing 220-250 g. All animals were subjected to two weeks of observation prior to the experiment to exclude infection. Rats were housed in standard cages, 5 individuals in each. The animals stayed in a room with a temperature of 22±2°C, air humidity 60-70% and in natural lighting (12/12 in a day/night cycle). The animals had free access to water and standard laboratory feed ad libitum.

Diabetes was caused by a single intraperitoneal administration of alloxan at a dose of 170 mg/kg bw. The development of diabetes was observed on the basis of the main symptoms (polydipsia, polyphagia), glycosuria and hyperglycemia. Animals with high glucose levels of at least 14 mmol/L were selected for the experiment.

Animals were randomized into 8 groups of 8 individuals each. The first group, the control group, was formed from healthy animals without diabetes. The control group (healthy animals) received NaCl 1 ml/kg; Group 2 - DM + Lupeol, at a dose of 100 mg/kg; Group 3 - DM + Betulonic acid, at a dose of 100 mg/kg; Group 4 - DM + 3,28-betulin diacetate, at a dose of 100 mg/kg; Group 5 - DM + Betulinic acid, at a dose of 50 mg/kg; Group 6 - DM + Betulinic acid, at a dose of 100 mg/kg; Group 7 - DM + Betulin, at a dose of 50 mg/kg; Group 8 - DM + Betulin, at a dose of 100 mg/kg. All test compounds were administered intragastrically as a suspension in a 2% starch solution for 30 days.

Alloxan administration has been shown to cause pronounced destructive changes in the rat pancreatic island apparatus. In microscopic examination, signs of necrotic β-cell death were observed in most pancreatic islets, in most cases cytoplasmic vacuolization, reduced testicular size, chromatin condensation and apoptosis were observed. Inflammatory changes were also observed, which were demonstrated by lymphocytic infiltration along the periphery of the islets. Morphometric results showed that the number, area and diameter of islets in diabetic animals were statistically significantly reduced by 1.5-2 times compared to control values.

In animals treated with betulin and betulinic acid at doses of 50 mg/kg/day for 30 days, pathological changes persisted in the islet pancreatic apparatus. There is a tendency to increase the area of Langerhans islets and their number. The islet diameter increased significantly (by 10-15%) and the number of β-cells in the islets (by 25-35%) compared to the untreated group.

The introduction of betulin and betulinic acid at doses of 100 mg/kg/day for 30 days was accompanied by a significant improvement in the morphological picture of the pancreas compared to the untreated group. In particular, an increase in the surface of the islands, their diameter, and an increase in the number of β-cells in the composition of the islands was noted.

The task set out was solved as a result of developing a dietary supplement composition using a pharmaceutical carrier is selected from the group consisting of polyvinylpyrrolidone, cellulose derivatives, including hydroxypropylmethylcellulose and hydroxypropylcellulose, cyclodextrin, gelatin, hypromellose phthalate, sugars and polyhydric alcohols, calcium sulfate dihydrate, dibasic calcium phophate, sorbitol, mannitol, anhydrous lactose, dextrose, acacia, tragacanth, ethylcellulose, methylcellulose, polyvinyl alcohol, sodium carboxymethylcellulose, silicon dioxide, polyethylene glycol 400, polyethylene glycol 4000, starch, porous silica carriers, aluminum silicate, calcium silicate, sugars, magnesium stearate, cellulose, calcium phosphate and their combinations, from which a capsule will be prepared, into which a biologically active substance from the lupane triterpenoid group in the amount of: 500 or 800 mg of substance in one capsule. They correspond to the sizes "0" and "00", which is a size that allows easy swallowing.

## Claims

1. A preparation with hypoglycemic effect used adjunctively in patients with diabetes, and having an impact on the activity of carbohydrate metabolizing enzymes and on the expression profile of the enzyme genes involved in metabolism, containing as an active substance a compound or compounds from the group of triterpenoid lupane isolated from birch bark Betula pendula Roth, **characterized in that** lupeole contains 0.5-24.5% by w/w, betulin contains 0.5-24.5% w/w, betulinic acid contains 0.5-24.5% w/w, betulonic acid contains 0.5-24.5% w/w.

2. The preparation as in claim 1 **characterized in that** the active ingredients enclosed in a gelatin capsule are in an amount of 500 or 800 mg.
